Europäisches Patentamt

European Patent Office

Office européen des brevets

(11) Publication number: **0 309 177**
A1

## EUROPEAN PATENT APPLICATION

(21) Application number: 88308661.3

(22) Date of filing: 19.09.88

(51) Int. Cl.4: **C07C 43/04 , C07C 41/06**

(30) Priority: 25.09.87 US 100874

(43) Date of publication of application:
29.03.89 Bulletin 89/13

(84) Designated Contracting States:
BE DE FR GB IT NL SE

(71) Applicant: **MOBIL OIL CORPORATION**
**150 East 42nd Street**
**New York New York 10017(US)**

(72) Inventor: **Bell, Weldon Kay**
**428 Burd Street**
**Pennington New Jersey 08534(US)**
Inventor: **Haag, Werner Otto**
**38 Pine Knoll Drive**
**Lawrenceville New Jersey 08648(LU)**

(74) Representative: **Colmer, Stephen Gary**
**Patent Department c/o Mobil Services**
**Company Limited Mobil Court 3 Clements**
**Inn**
**London WC2A 2EB(GB)**

(54) **Ether synthesis with zeolite beta.**

(57) A catalytic process is provided for the manufacture of C5 + ethers by reacting a linear monoolefin with a primary or secondary alcohol in the presence of a zeolite such as Zeolite Beta.

EP 0 309 177 A1

## ETHER SYNTHESIS WITH ZEOLITE BETA

This invention is concerned with the manufacture of ethers from lower aliphatic alcohols and linear olefins. In particular it is concerned with the manufacture of ethers by the catalytic reaction of linear monoalkenes with primary or secondary alcohols having up to four carbon atoms. It is further concerned with the manufacture of methyl sec-amyl ether or the like.

Numerous efforts have been made to synthesize ethers from alkanols and alkenes, particularly using acid catalysts. For instance, U.S. 4,262,145 ( Selwitz et al) discloses the catalytic reaction of a branched olefin such as isobutylene, 2-methylpentene-2, 2-methylbutene-2 and 2,3-dimethyloctene-2 with a lower alkanol such as methanol to form a mixed ether such as methyl tert-butyl ether. The catalyst disclosed in silicotungstic acid.

There is a need for an efficient catalytic process to manufacture ethers from linear monoolefins, thereby augmenting the supply of high-octane blending stock for gasoline. The C5+ lower molecular weight ethers, such as methyl sec-amyl ether, are in the gasoline boiling range and known to have a high blending octane number. In addition, by-product propylene and butylenes are usually available in a fuels refinery. The petrochemicals industry also produces linear olefin streams in the $C_3$ to $C_{15}$ molecular weight range, and the conversion of such streams or fractions thereof to ethers can provide products useful as solvents and as blending stocks for fuels.

This invention provides a catalytic process for selectively reacting one or more linear monoolefins with a primary or secondary lower molecular weight alcohol to form the corresponding C5+ ethers. The active acidic catalyst component may consist essentially of crystalline silicate having a pore size of about 7-8 A.U. Of the crystalline silicates, those preferred include crystalline zeolites having a silica to alumina mol ratio greater than about 12. In a particularly preferred embodiment, methanol and C4+ mono-olefin are reacted to selectively form methyl sec-alkyl ethers.

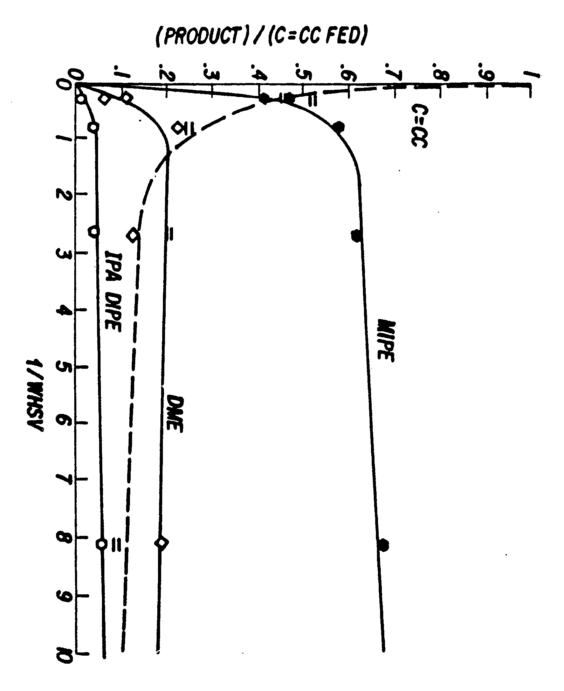

The single figure is a plot for a continuous reactor run with varying space velocity showing methyl alkyl ether production with Zeolite Beta catalyst.

The process of this invention is based on the discovery that linear monoolefins, under the proper reaction conditions, react in the presence of a solid insoluble acid catalyst with a low molecular weight primary or secondary monohydric alcohol to selectively produce the ethers.

Linear monoolefins and mixtures thereof useful in the process of this invention include, broadly, those having 3 to 15 carbon atoms, and these have the structure:

$$R_1\text{-}CH = CH\text{-}R_2$$

wherein $R_1$ and $R_2$ individually are hydrogen or n-alkyl groups and the total carbon atoms is at least 4. In a preferred embodiment of the present invention, the preferred linear olefins are those having 4 to 5 carbon atoms.

Although the hydrocarbon feed to the process may be substantially pure linear olefin (i.e. greater than 90 wt% of olefin), it is a feature of the invention that the reaction proceeds well in the presence of paraffin. For example, the by-product $C_4$ stream recovered from catalytic cracking in a typical petroleum refinery and containing 20-80 wt% n-butenes mixed with butanes can be used as feed. Branched olefins such as isobutylene may be present up to about 10 mol% of the total olefin content of the feed. Under the process conditions of this invention, branched olefins are very rapidly converted to higher boiling oxygenates or oligomers which if desired, may readily be separated from the feed or the ether products. Small amounts of dienes, such as up to about 2 mol% of the olefin content of the hydrocarbon feed, also may be present.

The alcohol to be reacted with the linear olefin is any primary or second alcohol having 1 to 4 carbon atoms. These include the primary alcohols methanol, ethanol, n-propanol, n-butanol and isobutanol; and the secondary alcohols isopropanol and sec-butanol. The primary alcohols are preferred, with methanol being particularly preferred. Accordingly, the ether reaction product may have a total 5 to 15 or more carbon atoms.

The process of this invention may be conducted batchwise; however, it is generally advantageous and preferred to conduct the process as a continuous operation. Since the reaction is exothermic, temperature control is facilitated by a continuous cascade operation with two or more reactors in sequence and with interstage cooling. Operable reaction conditions are given in Table A. The weight hourly space velocity (WHSV) referred to in Table A and elsewhere herein, unless explicitly stated to be otherwise, is based on total reactants, i.e. weight of linear olefin plus alcohol, divided by the total weight of active, binder-free, undiluted solid acid catalyst per hour. The corresponding contact times, of course, apply to batch conversions.

TABLE A

| REACTION CONDITIONS | | | | |
|---|---|---|---|---|
| | Mol Ratio Alcohol/Olefin | Temp., °C | Total Pressure kPa (Atm) | WHSV Hr⁻¹ |
| Broad | 0.1 - 10 | 50 - 300 | 101 - 30400 (1.0 - 300) | 0.05 - 50 |
| Preferred | 0.3 - 3 | 80 - 250 | 506 - 202600 (5 - 200) | 0.2 - 20 |
| Most Preferred | 0.5 - 2 | 100 - 210 | 1013 - 10130 (10 - 100) | 0.5 - 10 |

The principal ether product or products produced depends on the linear olefin and the alcohol charged. In the case of methanol reacted with butene-1 or the cis- or trans-butene-2, methyl sec-butyl ether is formed. In brief, the ethers formed are those predicted by the Markovnikov rule for addition to the double bond of the linear olefin. In the case of the higher molecular weight linear monoolefins, or mixtures of olefins, the principal reaction product is a mixture of such ethers.

By-products formed in the conversion includes water and ether resulting from the autocondensation of the alcohol charged. Other by-products may include alcohol resulting from the hydration of the linear monoolefin, and the ether formed by the self-condensation of the latter alcohol. A small amount of hydrocarbon may also be formed, believed to be the oligomer of the olefin charged. This hydrocarbon by-product appears to account for substantially less than 5 wt% of the total olefin converted under moderate temperatures, such as at a temperature not higher than about 160° C.

This invention is illustrated by the following examples. Many modifications and variations of the process as illustrated by the examples can be made without departing from the spirit and scope of the invention. In the following examples reactants, products and by-products will be abbreviated as follows: methanol (MeOH), dimethyl ether (DME); isopropyl alcohol (IPA); methyl isopropyl ether (MIPE); diisopropyl ether (DIPE), sec-amyl methyl ether (SAME).

Example 1

Equimolar mounts of methanol and 2-pentene are reacted continuously in a fixed bed, tubular reactor at about 150-152° C and 6630 kPa (950 psig) over solid catalyst particles. The zeolite beta comprises self-bound extrudate (1.6 mm diam.) diluted with silica sand particles to a catalyst concentration of 0.2 gm/cc. The sulfonic acid catalyst is standard "Amberlyst 15" beads (0.5 mm diam.), diluted to 0.25 gm/cc. The results of four comparative runs are tabulated below:

TABLE 1

| CONVERSION OF METHANOL AND 2-PENTENE FEED TO SEC-AMYL METHYL ETHER (SAME) AND DIMETHYL ETHER (DME) | | | | |
|---|---|---|---|---|
| CATALYST | ZEOLITE BETA | | SULFONIC ACID RESIN (AMBERLYST-15) | |
| Run | 1A | 1B | 1C | 1D |
| Time on stream (Hours) | 4 | 5 | 5 | 7 |
| WHSV (based on olefin and active catalyst) | 4.03 | 16.36 | 3.93 | 3.93 |
| Conversion (Based on 2-pentene) | 15.2 | 5.0 | 6.3 | 8.6 |
| Ether Selectivity Moles (SAME:DME) | 3.0 | 4.7 | 0.32 | 0.27 |
| Reaction Effluent | Wt % | | | |
| Water | 0.9 | 0.8 | 3.6 | 0.4 |
| MEOH | 23.6 | 31.9 | 9.7 | 8.2 |
| DME | 2.0 | 0.3 | 8.4 | 13.9 |
| 2-C5 = | 59.0 | 62.9 | 71.8 | 68.7 |
| SAME | 13.4 | 3.1 | 6.0 | 8.3 |
| HYDRO CARBON | 1.1 | 1.0 | 0.6 | 0.6 |

These runs clearly demonstrate the unexpected improved selectivity of zeolite beta as compared to acidic resin. The synthesis of sec-amyl methyl ethers (SAME) from an equimolar stoichiometric mixture of methanol plus 2-pentene shows that beta gives a significantly higher selectivity of SAME vs DME, as compared to the resin. Data and test conditions are summarized in Table 1. At the test conditions, the beta catalyst gives about a ten times higher molar ratio of SAME to DME than Amberlyst-15.

Example 2

Improved selectivity of zeolite Beta as compared to other acid catalysts is found for synthesis of isopropyl ether ether (IPEE) as compared to diethyl ether (DEE) from equimolar feed of ethanol (EtOH) and propylene (C = CC). Here, either C = CC and EtOH form IPEE: or 2 EtOH form DEE + $H_2O$; followed by the subsequent reaction of $H_2O$ + C = CC forming isopropyl alcohol (IPA): and IPA + C = CC forming diisopropyl ether (DIPE). With Table 2 (Zeolite Beta) and Table 3 (acid resin) clearly show that zeolite Beta has superior selectivity for the desired formation of the mixed ether IPEE as opposed to the competing parallel formation of DEE. At the test conditions, the Beta catalyst gives about a 50% higher molar ratio of IPEE to DEE than Amberlyst-15 resin. The process conditions of Example 1 are repeated except that equimolar ethanol/propylene feed is converted over zeolite beta catalyst (65%) extruded with 35% alumina binder and diluted with sand to provide 0.25 $gm/cm^3$ active catalyst concentration

TABLE 2

| HR ON STREAM | 2 | 4 | 5 | 23 |
|---|---|---|---|---|
| WHSV (based on olefin and active catalyst) | 3.74 | 1.86 | 0.93 | 0.31 |
| CONVERSION % C = CC | 13.9 | 26.1 | 26.8 | 56.7 |
| MOLAR SELECTIVITY: | | | | |
| IPEE/DEE | 4.45 | 4.87 | 5.01 | 4.26 |
| REACTION EFFLUENT | WT PCT | | | |
| WATER | 0.2 | 0.2 | 0.3 | 0.4 |
| C = CC | 37.2 | 31.8 | 33.9 | 18.8 |
| ETOH | 47.9 | 41.1 | 36.0 | 21.9 |
| IPA | 0.3 | 0.6 | 0.7 | 1.8 |
| DEE | 2.3 | 3.8 | 4.2 | 9.2 |
| IPEE | 12.1 | 22.2 | 24.8 | 46.6 |
| DIPE | 0.1 | 0.2 | 0.1 | 1.3 |

TABLE 3

| CATALYST CONC. gm/cc | 0.06 | 0.06 | 0.06 | 0.25 | 0.25 | 0.25 |
|---|---|---|---|---|---|---|
| HR ON STREAM | 2 | 4 | 8 | 3 | 5 | 6 |
| WHSV (based) on active catalyst | 23.30 | 11.29 | 5.41 | 5.62 | 2.88 | 1.43 |
| CONVERSION % C = CC | 17.9 | 21.2 | 46.0 | 60.6 | 73.5 | 78.3 |
| MOLAR SELECTIVITY: | | | | | | |
| IPEE/DEE | 2.51 | 2.76 | 3.41 | 3.18 | 3.19 | 3.29 |
| PRODUCT STREAM WT PCT | | | | | | |
| WATER | 0.3 | 0.1 | 0.3 | 0.3 | 0.3 | 0.4 |
| C = CC | 29.3 | 44.2 | 24.5 | 16.9 | 11.7 | 9.6 |
| ETOH | 53.8 | 25.4 | 25.3 | 19.5 | 10.1 | 7.9 |
| IPA | 1.1 | 1.8 | 3.4 | 4.8 | 5.1 | 5.4 |
| DEE | 3.8 | 6.5 | 8.9 | 11.9 | 14.6 | 14.8 |
| IPEE | 11.5 | 21.4 | 36.2 | 45.1 | 55.3 | 57.9 |
| DIPE | 0.1 | 0.5 | 1.4 | 2.9 | 4.0 | |

Example 3

A long-term reaction is conducted to demonstrate reaction kinetics for the etherification process using zeolite beta catalyst. In this example the catalyst used is 65 wt% zeolite beta with 35 wt% alumina binder, using acid zeolite having a silica to alumina ratio of 37:1. The results are shown in Table IV.

## TABLE IV

| CONTINUOUS REACTOR, EXAMPLE 3 | | | | | |
|---|---|---|---|---|---|
| Catalyst - 18.2 grams Zeolite Beta Extrudate | | | | | |
| Hr on Stream | 2.5 | 24.3 | 28.0 | 48.0 | 50.2 |
| Temp, °C | 163 | 160 | 161 | 163 | 163 |
| WHSV | 3.70 | 0.12 | 1.23 | 0.37 | 3.70 |
| CONVERSION (%) BASED ON PRODUCTS | | | | | |
| Propylene | 54.18 | 90.75 | 74.65 | 79.08 | 47.63 |
| Methanol | 67.02 | 85.80 | 85.07 | 90.06 | 65.24 |
| PRODUCT STREAM, WT% | | | | | |
| Water | 1.49 | 5.24 | 2.10 | 1.05 | 0.81 |
| Propylene | 25.71 | 4.61 | 13.24 | 11.97 | 31.81 |
| Methanol | 14.55 | 6.62 | 7.26 | 4.23 | 13.72 |
| DME | 6.77 | 10.28 | 13.13 | 8.09 | 4.58 |
| IPA | 2.06 | 3.59 | 4.02 | 4.36 | 1.55 |
| MIPE | 46.61 | 59.40 | 53.47 | 62.72 | 44.81 |
| DIPE | 1.72 | 7.16 | 5.31 | 5.57 | 1.72 |
| Other* | 1.09 | 3.09 | 1.47 | 2.01 | 0.99 |

* by-products, probably propylene oligomers and their oxygenated derivatives.

The principal reaction products from propylene and methanol are methyl isopropyl ether and dimethyl ether. This is clearly brought out with the Zeolite Beta catalyst, and with the graphical representation of the drawing.

A particularly preferred embodiment of this invention utilizes a hydrocarbon feed consisting of about 20 to 100 wt% linear alkene and reacts this feed with methanol. The product formed is separated into a fraction comprising methyl alkyl ether useful as a gasoline blending component. Unreacted alkene, methanol, and by-product water, individually or in combination, can be combined with fresh feed and recycled to the process. In another embodiment of this invention, it is contemplated to feed dimethyl ether and about stoichiometric water to form the methanol in situ.

The crystalline catalyst is a porous, crystalline metallosilicate solid having a highly ordered, robust three-dimensional framework structures as evidenced by well-defined and reproducible X-ray diffraction patterns which are distinctive for the different framework structures. The ordered structure contains intracrystalline micropores, i.e. pores of molecular dimensions, regularly disposed in the crystal lattice and readily distinguishable from the much larger extracrystalline pores formed by agglomerates of microcrystals. Because of their intracrystalline nature, these pores are very uniform and, when free of occluded matter, selectively sorb only those molecules having a critical diameter that can be accommodated by the pore size of the particular zeolite.

Crystalline zeolite beta has a pore size of about 7-8 A.U., as evidenced by a sorption capacity for cyclohexane. Zeolite Beta is preferred because of its high activity and its high selectivity for producing methyl alkyl ethers with reduced formation of byproduct dimethyl ether. Crystalline Zeolite Beta and its conventional preparation are disclosed in U.S. Patent 3,308,069, incorporated herein by reference. It has an X-ray diffraction pattern which distinguishes it from other known crystalline silicates, and a constrained access to molecules larger than normal paraffins. A simple determination of the "Constraint Index", or C.I., as herein defined may be made by passing continuously a mixture of an equal weight of normal hexane and 3-methylpentane over a small sample, approximately one gram or less, of mineral (in the hydrogen form) at atmospheric pressure according to the procedure described in US Patent No. 4,016,218.

The Constraint Index approximates the ratio of the cracking rate constants for the two hydrocarbons. Zeolites of the Beta type have a Constraint Index of about 0.5 to 2.C.I. values for some typical zeolites are shown in below.

EP 0 309 177 A1

TABLE V

| Constraint Indices of Zeolites | |
| --- | --- |
| CAS | C. I. |
| ZSM-5 | 8.3 |
| ZSM-12 | 2 |
| ZSM-38 | 2 |
| Beta | 0.6 |
| REY | 0.4 |

The above-described Constraint Index is an important property of the zeolite useful in the instant invention. The very nature of this parameter and the recited technique by which it is determined, however, admit of the possibility that a given zeolite can be tested under somewhat different conditions and thereby have different Constraint Indices. The Constraint Index seems to vary somewhat with severity of operation (conversion) and the presence or absence of binders. Therefore, it will be appreciated that it may be possible to so select test conditions to establish more than one value for the Constraint Index of the zeolite.

The crystalline silicates described herein are preferably used in the hydrogen form, although in some instances it is contemplated that some advantage may accrue from ion-exchange with divalent or high-valent metal cations. Also, the crystals may be composited with a binder such as alumina, silica-alumina, clay, or other material used for such purpose. Such composites, in general, contain 10 to 90 wt% of zeolite, preferably 20 to 80 wt%.

**Claims**

1. A process for producing ethers from a hydrocarbon feed consisting of one or more linear monoolefins having at least four carbon atoms by reaction with a primary or secondary alcohol having 1 to 4 carbon atoms, which process comprises:
passing said hydrocarbon feed and alcohol to a reaction zone that contains a catalytic amount of crystalline silicate having the structure of Zeolite Beta
and contacting said cofed mixture with said silicate catalyst under a combination of conditions effective to form said ethers, said conditions including 101 to 30400 kPa pressure, a temperature of 50°C to about 300°C, and a WHSV of 0.05 to 50.

2. The process described in Claim 1 wherein said one or more linear monoolefins have 4 to 5 carbon atoms.

3. The process described in Claim 1 or 2 wherein said alcohol is methanol or ethanol.

4. The process of any one of the preceding claims wherein methanol is reacted with linear amylene to produce predominantly methyl sec-amyl-ether.

5. The process for the manufacture of C5+ ether from lower alkanol and at least one $C_3$-$C_{15}$ linear olefin, which process comprises:
preparing a mixture of linear olefin and 0.1 to 10 mols of alkanol per mol of olefin,
contacting said mixture with a solid insoluble acidic catalyst comprising material having the structure of Zeolite Beta, said contacting being effected under a combination of conditions effective to selectivity form said ether, said conditions including 101 to 30400 kPa pressure, a temperature of 50°C to 300°C, and a WHSV of 0.5 to 50, and
recovering from said contacted mixture a product comprising C5+ ether.

6. The process of claim 5 wherein said zeolite is in the hydrogen form.

7. The process of claim 5 wherein said lower alkanol consists essentially of ethanol, said olefin comprises propylene, and said ether comprises ethyl isopropyl ether.

8. A process for the manufacture of ethyl isopropyl ether from ethanol and a $C_3$ hydrocarbon fraction that contains about 20 to 100 wt% of propylene, which process comprises:
preparing a mixture of said hydrocarbon fraction and 0.1 to 10 mols of ethanol per mol of propylene contained in said fractions,
contacting said mixture with a solid acidic catalyst comprising crystalline a metallosilicate having a rigid three-dimensional framework having a pore size of about 7 to 8 .AU., said contacting being at a temperature of 50° C-300° C, a total pressure of 101 to 30400 kPa, and a WHSV of 0.05-50, and
recovering from said contacted mixture a fraction comprising ethyl isopropyl ether.--

9. The process of claim 8 wherein said acidic catalyst consists essentially of Zeolite Beta.--

10. The process of claim 9 wherein said acidic catalyst is in the hydrogen form.--

11. The process of claim 10 wherein said acidic catalyst constis essentially of Zeolite Beta.--

12. A process for the manufacture of methyl sec-amyl ether from methanol and a hydrocarbon fraction that contains about 20 to 100 wt% of 2-pentene, which process comprises:

preparing a mixture of said hydrocarbon fraction and 0.1 to 10 mols of methanol per mol of 2-pentene contained in said fraction,

contacting said mixture with a solid acidic catalyst comprising crystalling a metallosilicate having a rigid three-dimensional framework having a pore size of about 7 to 8 A.U. and a Constraint Index of about 0.5 to 2, said contacting being at a temperature of 50° C-300° C, a total pressure of 101 to 30400 kPa, and a WHSV of 0.05-50, and

recovering from said contacted mixture a fraction comprising methyl sec-amyl ether.--

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 213 884 (MOBIL OIL) <br> * Claims; example 3 * | 1-3,5,6 | C 07 C 43/04 <br> C 07 C 41/06 |
| A | | 4,7-12 | |
| D,A | US-A-4 262 145 (C.M. SELWITZ) <br> * Whole document * | 1-12 | |
| A | US-A-4 175 210 (C.M. SELWITZ) <br> * Whole document * | 1-12 | |

TECHNICAL FIELDS
SEARCHED (Int. Cl.4)

C 07 C 41/00
C 07 C 43/00

The present search report has been drawn up for all claims

| Place of search | Date of completion of the search | Examiner |
|---|---|---|
| THE HAGUE | 29-11-1988 | WRIGHT M.W. |

EPO FORM 1503 03.82 (P0401)